# EUROPÄISCHE PATENTSCHRIFT

(11) **EP 2 276 429 B1**
(45) Veröffentlichungstag und Bekanntmachung des Hinweises auf die Patenterteilung: **12.03.2014**
(21) Anmeldenummer: 09728953.2
(22) Anmeldetag: 19.01.2009
(51) Int. Cl.: A61F 2/78

(54) **ÜBERGANGSADAPTER**
CONNECTION ADAPTER
ADAPTATEUR DE TRANSITION

(30) Priorität: 03.04.2008 DE 102008000977
(43) Veröffentlichungstag der Anmeldung: 26.01.2011
(73) Patentinhaber: Orthodynamics GmbH, 23556 Lübeck (DE)
(72) Erfinder: GRUNDEI, Hans, 23558 Lübeck (DE)
(74) Vertreter: Hughes, Andrea Michelle
(86) Internationale Anmeldenummer: PCT/EP2009/050552
(87) Internationale Veröffentlichungsnummer: WO 2009/121636

(56) Entgegenhaltungen:
- WO-A-2007/018904
- GB-A- 2 411 841

## Beschreibung

Die vorliegende Erfindung betrifft einen Übergangsadapter zwischen einem starren transkutanen Implantat, welches intrakorporal in einem Femurstumpf verankerbar ist, und einem Teil eines extrakorporalen orthopädischen Kniegelenks.

Ein derartiges transkutanes Implantat ist bekannt aus der DE 198 26 638 C2. Der darin offenbarte Adapter für ein exoprothetisches Standardteil ist mit einem proximalen Stielteil in einen Röhrenknochenstumpf setzbar, wobei das Stielteil zumindest teilweise mit einer offenmaschigen, dreidimensionalen Raumnetzstruktur bedeckt ist und an seinem distalen Ende mit einer Koppelungseinrichtung für das exoprothetische Standardteil versehen ist. Die offenmaschige, dreidimensionale Raumnetzstruktur, die auch als interkonnektierend bezeichnet wird, ermöglicht ein Ein-, Durch-, Hinter- und Umwachsen von natürlichem Knochenmaterial während der Einheilphase, so dass der Stielteil nach relativ kurzer Zeit jedenfalls im Hinblick auf den Substratfluss im Röhrenknochen integriert wird und eine extrem stabile Sekundärfixation gewährleistet wird. Das distale Ende des Implantates tritt aus dem Gliedmaßenstumpf heraus und bietet eine Ankoppelungsmöglichkeit für ein exoprothetisches Standardteil, beispielsweise ein extrakorporales orthopädisches Gelenk.

Die dadurch erzielte Unmittelbarkeit der Wahrnehmung der externen Belastungen im Skelett des Patienten wird auch als Osteoperzeption bezeichnet. Diese gestattet eine vollkommen andersartige Wahrnehmung des Patienten mit beispielsweise einem Oberschenkel amputierten Bein.

Die Verbindung zwischen dem Adapter und dem natürlichen Knochen kann sich allerdings als so fest herausstellen, dass eben diese an sich gewünschte Festigkeit zu Problemen führen kann, wenn es nämlich um außergewöhnliche Kraftbelastungen geht. So ist es nicht von vornherein auszuschließen, dass eine extreme, beispielsweise Scherbelastung des künstlichen Kniegelenkes oder eine Belastung der Unterschenkelprothese in den intramedullär verankerten Adapter im Femurstumpf weitergeleitet wird und dort zu Brüchen führen kann. Hierzu ist in der EP 1 309 297 B1 vorgeschlagen worden, eine derartige Beinprothese so auszubilden, dass sie eine extrakorporal angeordnete Sollbruchstelle aufweist, welche durch Applikation einer Kraft vorbestimmter Größe das Prothesenteil abrechen lässt. Diese Art der beschriebenen Sollbruchstelle zielt ab auf eventuell auftretende Dreh- und Kippmomente, welche die Sollbruchstelle veranlassen können zu brechen.

Noch kritischer sind jedoch eventuell auftretende Torsionsmomente um die Femur-Tibiaachse. Treten zu hohe Torsionsmomente auf, so können diese zu einer Spiralfraktur oberhalb des im Femurstumpf verankerten Implantates führen. Im Endeffekt können derartige Torsionsmomente zu Schenkelhalsbrüchen führen. Dies gilt es selbstverständlich zu vermeiden.

Aus der GB 2411841 A ist ein Übergangsadapter mit den Merkmalen des Oberbegriffs des Anspruchs 1 bekannt. Die lösbare Verbindung zwischen dem ersten Koppelungsteil und dem zweiten Koppelungsteils wird durch eine Art Rutschkupplung hergestellt, die ab Überschreitung eines bestimmten Drehmomentes auskuppelt. Der Aufbau und die Konstruktionsweise dieses Übergangsadapters sind recht kompliziert. Von daher ist es die Aufgabe der vorliegenden Erfindung, den Aufwand für einen solchen Übergangsadapter der vorgenannten Art zu verringern.

Gelöst wird diese Aufgabe durch einen Übergangsadapter der eingangs genannten Art, wobei eine im Inneren des zweiten Koppelungsteils angeordnete freischaltende Sicherheitskupplung vorgesehen ist, die ein Torsionsmoment zwischen dem ersten Koppelungsteil und dem zweiten Koppelungsteil überträgt und bei Überschreiten eines einstellbaren Höchstwertes des Torsionsmomentes durchrutscht. In diesem Zusam-menhang wird unter einer freischaltenden Sicherheitskupplung eine solche verstanden, die nach Auslösung, das heißt nach der Trennung der Wirkverbindung zwischen dem ersten und zweiten Koppelungsteil, erst manuell wieder in den Zustand gebracht werden kann, um ihre bestimmungsgemäße Funktion auszuüben.

Überschreitet das Torsionsmoment den erwähnten Höchstwert, rutscht die Kupplung - wie ausgeführt - durch, das heißt, dass die Exoprothese unterhalb des Übergangsadapters um ihre Achse rotieren kann, womit eine Weiterleitung des hohen Torsionsmomentes in den Femurstumpf unterbleibt und die befürchteten Spiralfrakturen nicht auftreten. Nach einem solchen Fall wird der Übergangsadapter mit einfachen Handgriffen wieder in die Ausgangslage gebracht, so dass hierbei auch keine extremen Kosten anfallen.

Die erwähnte Sicherheitskupplung ist aufgebaut aus einer in dem zweiten Koppelungsteil fixierten Torsionsscheibe, von der mindestens ein Scherstift abragt, der in entsprechende Aufnahmen in einer Stirnseite einer Hülse greift, die ihrerseits mit dem ersten Koppelungsteil verbindbar ist.

Die zentrale Funktion der Sicherheitskupplung wird von dem wenigstens einen Scherstift wahrgenommen. Dieser ist so ausgelegt, dass er nach Überschreiten des Höchstwertes des Torsionsmomentes abschert, so dass dann keine form- und kraftschlüssige Verbindung mehr zwischen dem ersten und zweiten Koppelungsteil besteht. Mit anderen Worten wird der Scherstift nach Überschreiten des errechneten Höchstwertes des Torsionsmomentes abgeschert und die unterhalb des Übergangsadapters angeordnete Exoprothese kann um ihre Achse herumgedreht werden, ohne dass ein weiterer Schaden entstehen würde.

Der Höchstwert des Torsionsmomentes ist bevorzugt im Bereich von 5 bis 35 Nm einstellbar. Dies entspricht in etwa Belastungen, die bei einem 70 bis 130 kg schweren Patienten entstehen können.

Die Konstruktion ist vorteilhafter Weise so ausgelegt, dass das Torsionsmoment bis zum Höchstwert spielfrei übertragbar ist. Mit anderen Worten wird für einen optimalen Kraft- und Formenschluss zwischen dem Scherstift, der Torsionsscheibe und der erwähnten Hülse gesorgt.

Der Höchstwert des zu übertragenden Torsionsmomentes ist durch die Materialauswahl, die Formgebung oder durch die Anordnung von mehreren Scherstiften einstellbar.

So können die Scherstifte bevorzugt aus leicht verförmbarem Material wie beispielsweise Kupfer oder Messing bestehen. Hinsichtlich der Möglichkeit der Einstellung des Höchstwertes des zu übertragenden Torsionsmomentes ist die besonders bevorzugte Ausführungsform zu erwähnen, bei der mehrere Scherstifte mit unterschiedlich tiefen Einkerbungen versehen sind.

Hinsichtlich der Möglichkeit, den Höchstwert des zu übertragenden Torsionsmomentes durch die Anordnung von mehreren Scherstiften einzustellen, ist jene Ausführungsform erwähnenswert, bei der mehrere Scherstifte auf Kreissegmenten auf der Torsionsscheibe angeordnet sind. Die entsprechenden Aufnahmen in der Stirnseite der Hülse sind dann entsprechend angeordnet.

Besonders bevorzugt wird eine Ausführungsform, bei der der wenigstens eine Scherstift gegen einen anderen austauschbar ist. Dieses Merkmal ist dann von großer Bedeutung, wenn einmal der Höchstwert des zu übertragenden Torsionsmomentes überschritten worden ist und der oder die Scherstifte abgeschert worden sind. Die Austauschbarkeit der Scherstifte macht es dann nämlich möglich, den Übergangsadapter zu öffnen, die abgescherten Scherstifte zu entfernen und durch neue Scherstifte zu ersetzen. Die mit dem Austausch der Scherstifte verbundenen Kosten halten sich in Grenzen, das heißt es stellt keine absolute Katastrophe dar, wenn die Sicherheitskupplung bestimmungsgemäß ausgerückt ist.

Eine bevorzugte Weiterbildung sieht vor, dass an dem zweiten Koppelungsteil eine spannbare Spannfeder angelenkt ist, die im gespannten Zustand den Zusammenhalt der Komponenten gewährleistet und für die spielfreie Übertragung des Torsionsmomentes durch Aufeinanderpressen der Hülse auf die Torsionsscheibe unter Einschluss des wenigstens einen Scherstiftes sorgt. Die Spannkraft der Spannfeder sorgt für den schon erwähnten einwandfreien Kraft- und Formenschluss zwischen den Scherstiften und der Torsionsscheibe einerseits und den Scherstiften und der Hülse andererseits. Gleichzeitig stellt die Spannfeder ein leicht zu lösendes Element dar, um im Eventualfall die Scherstifte zu ersetzen. Besonders bevorzugt ist die Ausbildung der Spannfeder als Spannbügel.

Besonders bewährt hat sich im Übrigen eine Ausführungsform, bei dem das erste Koppelungsteil ein Doppelkonus und die Hülse eine Konushülse ist. Diese Ausführungsform gewährleistet in hohem Maße die Stabilität der Verbindungen bei gleichzeitig gegebener einfacher Lösbarkeit der Teile für eine eventuelle Revision.

Die Erfindung wird anhand eines Ausführungsbeispiels gemäß den Zeichnungsfiguren näher erläutert. Hierbei zeigt:
- Fig. 1: eine Seitenansicht des Übergangsadapters,
- Fig. 2: eine Explosionsdarstellung des Übergangsadapters gemäß Fig. 1,
- Fig. 3: die untere Stirnseite der Hülse des Adapters, und
- Fig. 4: die Oberseite der Torsionsscheibe des Adapters.

Nachfolgend sind gleiche Teile mit denselben Bezugszeichen versehen.

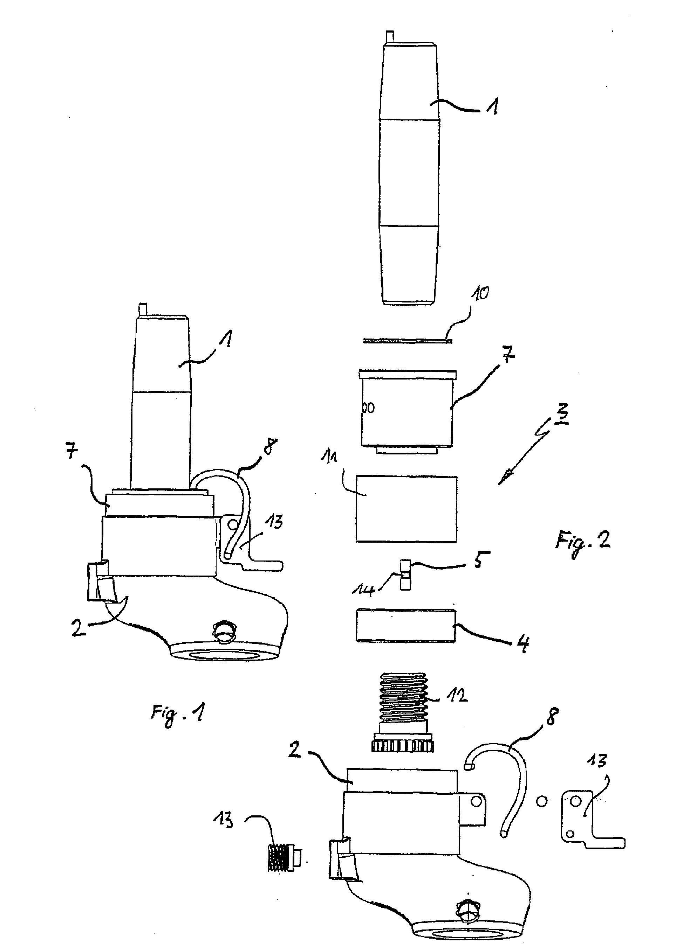

Einen ersten Überblick verschafft die Fig. 1. Diese zeigt die Seitenansicht des Übergangsadapters.

Dieser weist ein erstes Koppelungsteil 1 in Form eines Doppelkonus auf. Es dient zur Herstellung einer Verbindung mit dem transkutanen Implantat (nicht dargestellt), wohingegen der untere Konus des Doppelkonus 1 zur Herstellung einer Verbindung mit einer Konushülse 7 dient. Dabei durchdringt der Doppelkonus 1 einen Silikonring 10.

Das zweite Koppelungsteil 2 dient zur Verbindung mit einem extrakorporalen orthopädischen Kniegelenk, welches vorliegend nicht dargestellt ist.

Die Konushülse 7 steckt in einer Drehhülse 11. Zumindest ein Scherstift 5 stellt eine Verbindung zu einer Torsionsscheibe 4 her. Die Torsionsscheibe 4 dagegen wird in dem zweiten Koppelungsteil 2 fixiert.

Eine selbsthemmende Sicherungsschraube 12 durchdringt die Torsionsscheibe und dient zur Arretierung des konischen Klemmsitzes des Doppelkonus 1 in der Konushülse 7. Eine Sicherungsmadenschraube 13 ist gegen ein Lockern der Torsionsscheibe 4 vorgesehen. Die Teile werden durch eine Spannfeder in Form eines Spannbügels 8 zusammengehalten, welcher am zweiten Koppelungsteil 2 über einen Lagerbock 13 angelenkt ist. Hierbei drückt der Spannbügel 8 gegen den Silikonring 10 und weiter auf die Hülse 7, so dass die Teile einen stabilen Verbund eingehen.

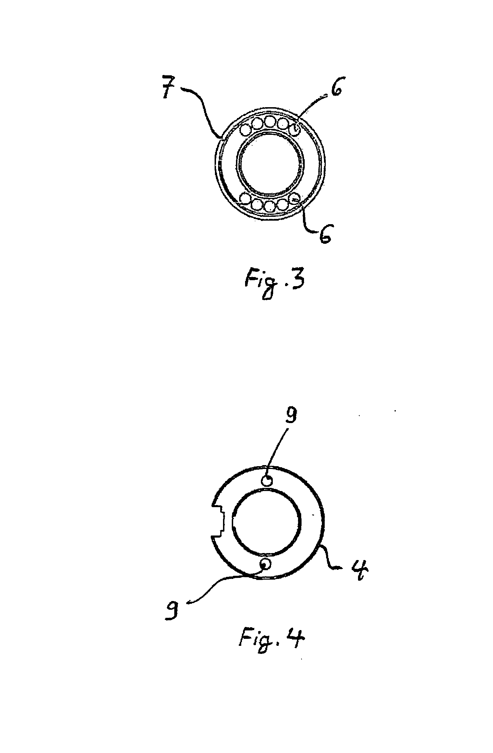

Kern des Übergangsadapters ist die Sicherheitskupplung 3, die insbesondere gebildet ist aus den Teilen Torsionsscheibe 4, Scherstift 5 und Hülse 7. Um dies verständlich zu machen, wird auf die Figuren 3 und 4 verwiesen.

Fig. 3 zeigt die untere Stirnseite der Hülse 7. Erkennbar ist eine Reihe von auf einem Kreissegment angeordneten Ausnehmungen oder Bohrungen 6. Diese sind so dimensioniert, dass in ihnen der oder die Scherstifte 5 formschlüssig aufgenommen werden können.

Gegenstück hierzu ist die Torsionsscheibe 4, von der in Fig. 4 die Oberseite gezeigt ist. Erkennbar sind hier zwei Ausnehmungen 9, in welche jeweils ein Scherstift 5 formschlüssig setzbar ist. Ein Torsionsmoment um die Hochachse des Übergangsadapters, das heißt um die Längsachse des Doppelkonus 1 herum, wird also durch die Torsionsscheibe 4 über die Scherstifte 5 auf die Hülse 7 übertragen. Der Scherstift 5 ist mit einer Einkerbung 14 versehen, durch deren Ausgestaltung der Abrisspunkt des Stiftes definiert werden kann, also jener Punkt, ab wann der Scherstift 5 bei einem bestimmten Torsionsmoment abgeschert wird. Nach einem Abscheren des Scherstiftes kann keine Übertragung des Torsionsmomentes vom zweiten zum ersten Koppelungsteil mehr stattfinden. Vielmehr kann sich das zweite Koppelungsteil (und damit das künstliche Kniegelenk) unter Vermittlung der Drehhülse 11 auf der Konushülse 7 drehen.

Im Falle der Abscherung von Scherstiften 5 kann der Übergangsadapter in einfacher Weise wieder hergestellt werden durch Öffnen des Spannbügels 8 und Herausnahme der abgescherten Scherstifte. Diese werden dann durch neue Scherstifte ersetzt und der Übergangsadapter durch Spannen des Spannbügels 8 wieder verschlossen.

## Patentansprüche

1. Übergangsadapter zwischen einem starren transkutanen Implantat, welches intrakorporal in einem Femurstumpf verankerbar ist, und einem Teil eines extrakorporalen orthopädischen Kniegelenks, aufweisend ein erstes Koppelungsteil (1) zur Verbindung mit dem transkutanen Implantat und ein zweites Koppelungsteil (2) zur Verbindung mit dem Kniegelenk, wobei das zweite Koppelungsteil (2) mit dem ersten Koppelungsteil (1) lösbar verbindbar ist; und
eine im Inneren des zweiten Koppelungsteils (2) angeordnete freischaltende Sicherheitskupplung (3), die ein Torsionsmoment zwischen dem ersten Koppelungsteil (1) und dem zweiten Koppelungsteil (2) überträgt und bei Überschreiten eines einstellbaren Höchstwertes des Torsionsmomentes durchrutscht; **gekennzeichnet dadurch, dass** die Sicherheitskupplung (3) aus einer in dem zweiten Koppelungsteil (2) fixierten Torsionsscheibe (4), von der mindestens ein Scherstift (5) abragt, der in entsprechende Aufnahmen (6) in einer Stirnseite einer Hülse (7) greift, die ihrerseits mit dem ersten Koppelungsteil (1) verbindbar ist, aufgebaut ist.

2. Übergangsadapter nach Anspruch 1, bei dem der Höchstwert des Torsionsmomentes im Bereich von 5 bis 35 Nm einstellbar ist.

3. Übergangsadapter nach Anspruch 1 oder 2, bei dem das Torsionsmoment bis zu dem Höchstwert spielfrei übertragbar ist.

4. Übergangsadapter nach Anspruch 1, bei dem der Höchstwert des zu übertragenden Torsionsmomentes durch die Materialauswahl, Formgebung oder Anordnung von mehreren Scherstiften (5) einstellbar ist.

5. Übergangsadapter nach Anspruch 1, bei dem die Scherstifte (5) aus leicht verformbarem Material bestehen.

6. Übergangsadapter nach Anspruch 5, bei dem das Material Kupfer ist.

7. Übergangsadapter nach Anspruch 5, bei dem das Material Messing ist.

8. Übergangsadapter nach einem der Ansprüche 4 bis 7, bei dem mehrere Scherstifte (5) mit unterschiedlich tiefen Einkerbungen versehen sind.

9. Übergangsadapter nach einem der Ansprüche 4 bis 8, bei dem mehrere Scherstifte (5) auf Kreissegmenten auf der Torsionsscheibe (4) angeordnet sind.

10. Übergangsadapter nach einem der Ansprüche 1 bis 9, bei dem der wenigstens einen Scherstift (5) gegen einen anderen austauschbar ist.

11. Übergangsadapter nach einem der Ansprüche 1 bis 10, bei dem an dem zweiten Koppelungsteil (2) eine spannbare Spannfeder (8) angelenkt ist, die im gespannten Zustand den Zusammenhalt der Komponenten gewährleistet und für die spielfreie Übertragung des Torsionsmomentes sorgt durch Aufeinanderpressen der Hülse (7) auf die Torsionsscheibe (4) unter Einschluss des wenigstens einen Scherstiftes (5).

12. Übergangsadapter nach Anspruch 11, bei dem die Spannfeder (8) als Spannbügel ausgebildet ist.

13. Übergangsadapter nach einem der Ansprüche 1 bis 12, bei dem das erste Koppelungsteil (1) ein Doppelkonus und die Hülse (7) eine Konushülse ist.

## Claims

1. Transition adapter between a rigid transcutaneous implant, which is fixable intracorporally in a femoral stump, and a part of an extracorporal orthopedic knee joint, comprising a first coupling part (1) for connecting with the transcutaneous implant and a second coupling part (2) for connecting with the knee joint, wherein the second coupling part (2) is removably connectable to the first coupling part (1); and an activatable safety coupling provided within the second coupling part (2), which transmits a torsional moment between the first coupling part (1) and the second coupling part (2) and in case of exceeding a settable maximum value of the torsional moment slips; **characterized in that** the safety coupling (3) is constructed from a torsion disc (4) fixed in the second coupling part (2), at least one shear pin (5) extending from the torsion disc, which engages corresponding receptions (6) in a front end of a sleeve (7), which for its part is connectable to the first coupling part (1).

2. Transition adapter according to claim 1, wherein the maximum value of the torsional moment is adjustable in the range of 5 to 35 Nm.

3. Transition adapter according to claim 1 or claim 2, wherein the torsional moment can be transferred up to the maximum value free of play.

4. Transition adapter according to claim 1, wherein the maximum value of the torsional moment to be transmitted is adjustable by choice of material, shaping or arrangement of a number of shear pins (5).

5. Transition adapter according to claim 1, wherein the shear pins (5) are formed of easily deformable material.

6. Transition adapter according to claim 5, wherein the material is copper.

7. Transition adapter according to claim 1, wherein the material is brass.

8. Transition adapter according to any one of claims 4 to 7, wherein a number shear pins (5) are provided with groovings of different depths.

9. Transition adapter according to any one of claims 4 to 8, wherein a number of shear pins (5) are arranged on circular segments of the torsion disc (4).

10. Transition adapter according to any one of claims 1 to 9, wherein the at least one shear pin (5) is exchangeable against another.

11. Transition adapter according to any one of claims 1 to 10, wherein a tensionable tension spring (8) is articulated on the second coupling part (2), which, in tensioned state, provides the cohesion of the components and serves to provide the play free transmission of the torsional moment by pressing the sleeve (7) onto the torsion disc (4) under inclusion of the at least one shear pin (5).

12. Transition adapter according to claim 11, wherein the tension spring (8) is provided as a bracket.

13. Transition adapter according to any one of claims 1 to 12, wherein the first coupling part (1) is a double conus and the sleeve (7) is a conus sleeve.

## Revendications

1. Adaptateur de transition entre un implant transcutané rigide, lequel peut être fixé en intracorporel dans un moignon fémoral, et une partie d'une articulation du genou orthopédique extracorporelle, comportant une première partie de couplage (1) en vue d'un raccordement à l'implant transcutané et une seconde partie de couplage (2) en vue d'un raccordement à l'articulation du genou, dans lequel la seconde partie de couplage (2) peut être reliée de manière amovible à la première partie de couplage (1) ; et un couplage de sécurité pouvant être activé prévu à l'intérieur de la seconde partie de couplage (2), qui transmet un moment de torsion entre la première partie de couplage (1) et la seconde partie de couplage (2) et dans le cas du dépassement d'une valeur maximale paramétrable du moment de torsion glisse ; **caractérisé en ce que** le couplage de sécurité (3) est conçu à partir d'un disque de torsion (4) fixé dans la seconde partie de couplage (2), au moins une goupille de cisaillement (5) s'étendant depuis le disque de torsion, lequel met en prise des réceptions correspondantes (6) dans une extrémité avant d'un manchon (7), lequel pour sa part peut être raccordé à la première partie de couplage (1).

2. Adaptateur de transition selon la revendication 1, dans lequel la valeur maximale du moment de torsion peut être réglable dans une plage de 5 à 35 Nm.

3. Adaptateur de transition selon la revendication 1 ou la revendication 2, dans lequel le moment de torsion peut être transféré jusqu'à la valeur maximale sans jeu.

4. Adaptateur de transition selon la revendication 1, dans lequel la valeur maximale du moment de torsion à transmettre est réglable par le choix du matériau, de la forme ou de l'agencement d'un certain nombre de goupilles de cisaillement (5).

5. Adaptateur de transition selon la revendication 1, dans lequel les goupilles de cisaillement (5) sont formées d'un matériau facilement déformable.

6. Adaptateur de transition selon la revendication 5, dans lequel le matériau est du cuivre.

7. Adaptateur de transition selon la revendication 1, dans lequel le matériau est du laiton.

8. Adaptateur de transition selon l'une quelconque des revendications 4 à 7, dans lequel un certain nombre de goupilles de cisaillement (5) sont munies de rainurages de différentes profondeurs.

9. Adaptateur de transition selon l'une quelconque des revendications 4 à 8, dans lequel un certain nombre de goupilles de cisaillement (5) sont disposées sur des segments circulaires du disque de torsion (4).

10. Adaptateur de transition selon l'une quelconque des revendications 1 à 9, dans lequel la au moins une goupille de cisaillement (5) peut être échangée par une autre.

11. Adaptateur de transition selon l'une quelconque des revendications 1 à 10, dans lequel un ressort de tension pouvant être tendu (8) est articulé sur la seconde partie de couplage (2), laquelle, dans un état tendu, permet la cohésion des composants et sert à fournir la transmission sans jeu du moment de torsion en pressant le manchon (7) sur le disque de torsion (4) sous inclusion de la au moins une goupille de cisaillement (5).

12. Adaptateur de transition selon la revendication 11, dans lequel le ressort de torsion (8) est fourni en tant que support.

13. Adaptateur de transition selon l'une quelconque des revendications 1 à 12, dans lequel la première partie de couplage (1) est un double conus et le manchon (7) est un manchon de conus.
